# EUROPEAN PATENT SPECIFICATION

(11) **EP 1 572 196 B1**
(45) Date of publication and mention of the grant of the patent: **13.08.2008**
(21) Application number: 03785782.8
(22) Date of filing: 09.12.2003
(51) Int. Cl.: A61K 31/40, A61K 31/216, A61K 31/195, A61K 31/192, A61P 3/00

(54) **COMBINATION OF A DPP-IV INHIBITOR AND A PPAR-ALPHA COMPOUND**
KOMBINATIONEN VON EINEM DPP-IV INHIBITOR UND EINEM PPAR- ALPHA AGONIST
COMBINAISION D'UN INHIBITEUR DE DPP-IV ET D'UN COMPOSE PPAR-ALPHA

(30) Priority: 10.12.2002 US 432192 P
(43) Date of publication of application: 14.09.2005
(73) Proprietor: Novartis AG, 4056 Basel (CH); Novartis Pharma AG, 1230 Vienna (AT)
(72) Inventor: WANG, Pei-Ran, Cambridge, MA 02139 (US)
(74) Representative: Morris, Clive Sydney
(86) International application number: PCT/EP2003/013963
(87) International publication number: WO 2004/052362

(56) References cited:
- WO-A-01/52825
- WO-A-01/60807
- WO-A-02/064094
- WO-A-02/083128
- WO-A-03/000181
- WO-A-03/004498
- WO-A-03/043985

## Description

The invention relates to a combination, such as a combined preparation or pharmaceutical composition, respectively, which comprises the dipeptidylpeptidase-IV (DPP-IV) inhibitor (*S*)-1-[(3-hydroxy-1-adamantyl)amino]acetyl-2-cyano-pyrrolidine, in free or pharmaceutically acceptable salt form, and a peroxisome proflierator-activated receptor α (PPARα), for simultaneous, separate or sequential use, especially in the prevention, delay of progression or treatment of conditions mediated by DPP-IV, in particular diabetes, more particular type 2 diabetes mellitus, conditions of impaired glucose tolerance (IGT), conditions of impaired fasting plasma glucose, metabolic acidosis, ketosis, arthritis, obesity, dyslipidemia and osteoporosis; the use of such combination for the preparation of a pharmaceutical preparation for the prevention, delay of progression or treatment of such conditions; the use of such combination for the cosmetic treatment of a mammal in order to effect a cosmetically beneficial loss of body weight; a method of prevention, delay of progression or treatment of conditions mediated by DPP-IV; a method of improving the bodily appearance of a warm-blooded animal.

DPP-IV is responsible for inactivating GLP-1. More particularly, DPP-IV generates a GLP-1 receptor antagonist and thereby shortens the physiological response to GLP-1. GLP-1 is a major stimulator of pancreatic insulin secretion and has direct beneficial effects on glucose disposal.

Non-insulin dependent diabetes mellitus (type 2 diabetes mellitus) is characterized by both increased peripheral insulin resistance and abnormal insulin secretion. At least three abnormalities of insulin secretion are recognized: in the first phase, insulin secretion is lost and in the second phase insulin is both delayed and inadequate in the face of elevated circulating glucose levels. Several metabolic, hormonal, and pharmacological entities are known to stimulate insulin secretion including glucose, amino-acids and gastrointestinal peptides. The Diabetes Control and Complications Trial (DCCT) has established that lowering of blood glucose is associated with decreases in the onset and progression of diabetic microvascular complications (see Diabetes Control and Complications Trial Research Group, N. Engl. J. Med., Vol. 329, pp. 977-986 (1993)). IGT is an impairment of glucose homeostasis closely related to type 2 diabetes mellitus. Both conditions convey a great risk of macrovascular disease. Therefore, one therapeutic focus is on optimizing and potentially normalizing glycemic control in subjects with type 2 diabetes mellitus, conditions of impaired fasting plasma glucose or IGT. Presently available agents need to be improved in order to better meet this therapeutic challenge.

The patent application WO-A-0152825 did already disclose the use of a DPP-IV inhibitor such as (*S*)-1-[(3-hydroxy-1-adamantyl)amino)acetyl-2-cyano-pyrrolidine, in combination with antidiabetic agents such as insulin signalling pathway modulators, like inhibitors of protein tyrosine phosphatases (PTPases), non-small molecule mimetic compounds and inhibitors of glutamine-fructose-6-phosphate amidotransferase (GFAT), compounds influencing a dysregulated hepatic glucose production, like inhibitors of glucose-6-phosphatase (G6Pase), inhibitors of fructose-1,6-bisphosphatase (F-1,6-BPase), inhibitors of glycogen phosphorylase (GP), glucagon receptor antagonists and inhibitors of phosphoenolpyruvate carboxykinase (PEPCK), pyruvate dehydrogenase kinase (PDHK) inhibitors, insulin sensitivity enhancers, insulin secretion enhancers, α-glucosidase inhibitors, inhibitors of gastric emptying, insulin, and α₂-adrenergic antagonists. Antidiabetic compounds such as glitazone type PPARγ agonists, non-glitazone type PPARγ agonists or dual PPARγ / PPARα agonists were also mentioned, but the antihyperlipidemic agents PPARα agonists were not mentioned.

The patent application WO-A-02083128 did already disclose the use of a DPP-IV inhibitor with any kind of combination partners such as anti-arthritis compounds, AIDS agents, transplantation agents, anorectic agents, agents to treat autoimmune disorders, osteoporosis, inflammatory bowel disease, infertility, anti-obesity agents. Among all the listed combination partners, peroxisome proliferators-activated receptor compounds (PPARα) have been mentioned.

The present invention relates to a combination which comprises a DPP-IV inhibitor which is (*S*)-1-[(3-hydroxy-1-adamantyl)amino]acetyl-2-cyano-pyrrolidine in free or pharmaceutically acceptable salt form, and a PPARα or the pharmaceutically acceptable salt of such a compound and optionally at least one pharmaceutically acceptable carrier; for simultaneous, separate or sequential use.

Preferably, the PPARα compound is a fibrate selected from the group consisting of fenofibrate, micronized fenofibrate, bezafibrate, gemfibrozil and ciprofibrate, or the pharmaceutically acceptable salts of such a compound and optionally at least one pharmaceutically acceptable carrier; for simultaneous, separate or sequential use, particularly in the prevention, delay of progression or treatment of conditions mediated by DPP-IV or PPARα, in particular conditions of IGT, conditions of impaired fasting plasma glucose, metabolic acidosis, ketosis, arthritis, obesity and osteoporosis, and preferably diabetes, especially type 2 diabetes mellitus, IGT, obesity and dyslipidemia. Such a combination is preferably a combined preparation or a pharmaceutical composition.

The present invention relates to the use of a DPP-IV inhibitor which is (*S*)-1-[(3-hydroxy-1-adamantyl)amino]acetyl-2-cyano-pyrrolidine, in free or pharmaceutically acceptable salt form, in combination with at least one further PPARα compound, or the pharmaceutically acceptable salt of such a compound,
for the manufacture of a medicament for the prevention, delay of progression or treatment of a disease selected from type 2 diabetes, impaired glucose tolerance, conditions of impaired fasting plasma glucose, obesity and dyslipidemia.

The DPP-IV inhibitor (S)-1-[(3-hydroxy-1-adamantyl)amino]acetyl-2-cyano-pyrrolidine (LAF237) is described in WO01/52825.

WO01/52825 specially discloses (S)-1 -{2-[5-cyanopyridin-2yl)amino]ethyl-aminoacetyl)-2-cyano- pyrrolidine or (S)-1 -[(3-hydroxy-1 adamantyl)amino]acetyl-2- cyano-pyrrolidine. LAF237 is specifically disclosed in Example 1 of WO 00/34241.

Preferred DPP-4 inhibitor is pyrrolidine, 1-[(3-hydroxy-1-adamantyl) amino] acetyl-2-cyano-, (S) (LAF237) of formula 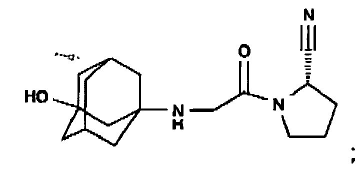 and pharmaceutical salts thereof.

The DPP-IV inhibitor can be used alone according to the present invention can be used in association with a carrier.

Comprised are likewise the corresponding stereoisomers as well as the corresponding polymorphs, e.g., crystal modifications, which are disclosed in the cited patent documents.

By the term "treatment" is understood the management and care of a patient for the purpose of combating the disease, condition, or disorder.

The term "prevention" means prophylactic administration of the combination to healthy patients to prevent the outbreak of the conditions mentioned herein. Moreover, the term "prevention" means prophylactic administration of such combination to patients being in a pre-stage of the conditions, especially diabetes, to be treated.

The term "delay of progression" used herein means administration of the combination, such as a combined preparation or pharmaceutical composition, to patients being in a pre-stage of the condition, especially diabetes, to be treated in which patients a pre-form of the corresponding condition is diagnosed.

The structure of the active agents identified by code nos., generic or trade names may be taken from the actual edition of the standard compendium "The Merck Index" or from databases, e.g., Patents International, e.g., IMS World Publications. Any person skilled in the art is fully enabled to identify the active agents and, based on these references, likewise enabled to manufacture and test the pharmaceutical indications and properties in standard test models, both *in vitro* and *in vivo.*

The compounds to be combined can be present as pharmaceutically acceptable salts. If these compounds have, for example, at least one basic center, they can form acid addition salts. Corresponding acid addition salts can also be formed having, if desired, an additionally present basic center. The compounds having an acid group (for example COOH) can also form salts with bases. For example, the compounds to be combined can be present as a sodium salt, as a maleate or as a dihydrochloride. The active ingredient or a pharmaceutically acceptable salt thereof may also be used in form of a hydrate or include other solvents used for crystallization.

Examples of PPARα compounds, include but are preferably, fenofibrate, micronized fenofibrate, bezafibrate, gemfibrozil and ciprofibrate, or a pharmaceutically acceptable salt of such a compound, and will be referred to hereinafter as COMBINATION PARTNER OF THE INVENTION. These PPARα compounds are disclosed in The Merck Index, 13th Edition, (2001), the contents of which is hereby incorporated by reference in its entirety as if set forth in full herein.

Thus in a first aspect, the present invention concerns a combination, such as a combined preparation or fixed combination, respectively, which comprises the dipeptidylpeptidase-IV (DPP-IV) inhibitor (*S*)-1-[(3-hydroxy-1-adamantyl)amino]acetyl-2-cyano-pyrrolidine (LAF237), in free or pharmaceutically acceptable salt form, and at least one peroxisome proflierator-activated receptor α (PPARα).

Preferably the invention concerns a combination for simultaneous, separate or sequential use

Preferably the combination is a pharmaceutical combination comprising the dipeptidylpeptidase-IV (DPP-IV) inhibitor (*S*)-1-[(3-hydroxy-1-adamantyl)amino]acetyl-2-cyano-pyrrolidine (LAF237), in free or pharmaceutically acceptable salt form, and at least one further PPARα compound or the pharmaceutically acceptable salt of such a compound and optionally at least one pharmaceutically acceptable carrier; for simultaneous, separate or sequential use.

Preferably the DPP-IV inhibitor is
(*S*)-1-[(3-hydroxy-1-adamantyl)amino]acetyl-2-cyano-pyrroiidine;
in free or pharmaceutically acceptable salt form, and the further PPARα compound is selected from the group consisting of fenofibrate, micronized fenofibrate, bezafibrate, gemfibrozil and ciprofibrate, or the pharmaceutically acceptable salt of such a compound.

Preferably the pharmaceutical combination is a combined preparation or a fixed combination.

Preferably the pharmaceutical combination is a combined preparation for simultaneous, separate or sequential use.

A combined preparation which comprises the dipeptidylpeptidase-IV (DPP-IV) inhibitor (*S*)-1-[(3-hydroxy-1-adamantyl)amino]acetyl-2-cyano-pyrrolidine (LAF237), in free or pharmaceutically acceptable salt form and at least one further COMBINATION PARTNER OF THE INVENTION and optionally at least one, i.e., one or more, e.g., two, pharmaceutically acceptable carrier for simultaneous, separate or sequential use is especially a "kit of parts" in the sense that the components, the DPP-IV inhibitor in free or pharmaceutically acceptable salt form and at least one further COMBINATION PARTNER OF THE INVENTION, can be dosed independently or by use of different fixed combinations with distinguished amounts of the components, i.e., at different time points or simultaneously. The parts of the kit of parts can then, e.g., be administered simultaneously or chronologically staggered, that is at different time points and with equal or different time intervals for any part of the kit of parts. Preferably, the time intervals are chosen such that the effect on the treated disease or condition in the combined use of the parts is larger than the effect which would be obtained by use of only any one of the components.

The present invention thus also relates to a kit of parts comprising
(a) an amount of the DPP-IV inhibitor (*S*)-1-1(3-hydroxy-1-adamantyl)aminolacetyl-2-cyano-pyrrolidine, or a pharmaceutically acceptable salt thereof in a first unit dosage form;
(b) an amount of at least one PPARα compound or the pharmaceutically acceptable salt thereof,
in the form of two or three or more separate units of the components (a) and (b).

Preferably the DPP-IV inhibitor is
(*S*)-1-[(3-hydroxy-1-adamantyl)amino]acetyl-2-cyano-pyrrolidine; and
and the further PPARα compound is selected from the group consisting of fenofibrate, micronized fenofibrate, bezafibrate, gemfibrozil and ciprofibrate

The invention furthermore relates to a commercial package comprising the combination according to the present invention together with instructions for simultaneous, separate or sequential use.

Preferably, there is at least one beneficial effect, e.g., a mutual enhancing of the effect of a DPP-IV inhibitor in free or pharmaceutically acceptable salt form, and at least one further COMBINATION PARTNER OF THE INVENTION, additional advantageous effects, less side effects, a combined therapeutical effect in a non-effective dosage of one or each of the components, and especially a synergism, e.g., a more than additive effect, between a DPP-IV inhibitor in free or pharmaceutically acceptable salt form, and at least one further COMBINATION PARTNER OF THE INVENTION.

The nature of conditions mediated by DPP-IV, especially diabetes, conditions of impaired fasting plasma glucose, and IGT, is multifactorial. Under certain circumstances, drugs with different mechanisms of action may be combined. However, just considering any combination of drugs having different mode of action but acting in the similar field does not necessarily lead to combinations with advantageous effects.

All the more surprising is the experimental finding that the combined administration of a DPP-IV inhibitor and at least one further COMBINATION PARTNER OF THE INVENTION results not only in a beneficial, especially a synergistic, therapeutic effect but also in additional benefits resulting from combined treatment such as a surprising prolongation of efficacy, a broader variety of therapeutic treatment and surprising beneficial effects on diseases and conditions associated with diabetes, e.g., less gain of weight or less cardiovascular side effects.

The term "synergistic" shall mean that the drugs, when taken together, produce a total joint effect that is greater than the sum of the effects of each drug when taken alone.

The diseases, disorders or conditions associated to diabetes, particularly type 2 diabetes mellitus, includes diabetic nephropathy, diabetic retinopathy and diabetic neuropathy, macular degeneration, coronary heart disease, myocardial infarction, diabetic cardiomyopathy, myocardial cell death, coronary artery diseases, peripheral arterial disease, stroke, limb ischemia, vascular restenosis, foot ulcerations, endothelial dysfunction and/or atherosclerosis.

Further benefits are that lower doses of the individual drugs to be combined according to the present invention can be used to reduce the dosage, for example, that the dosages need not only often be smaller but are also applied less frequently, or can be used in order to diminish the incidence of side effects. This is in accordance with the desires and requirements of the patients to be treated.

It can be shown by established test models and especially those test models described herein that the combination of a DPP-IV inhibitor, especially (*S*)-1-{2-[5-cyanopyridin-2-yl)amino]ethyl-aminoacetyl}-2-cyano-pyrrolidine (DPP728) or (*S*)-1-[(3-hydroxy-1-adamantyl)amino]acetyl-2-cyano-pyrrolidine (LAF237), and at least one further COMBINATION PARTNER OF THE INVENTION results in a more effective prevention or preferably treatment of conditions mediated by DPP-IV, in particular diabetes, especially type 2 diabetes mellitus, conditions of impaired fasting plasma glucose, and conditions of IGT.

The person skilled in the pertinent art is fully enabled to select a relevant animal test model to prove the hereinbefore and hereinafter indicated therapeutic indications and beneficial effects. The pharmacological activity may, for example, be demonstrated following essentially an *in vivo* test procedure in mice or in a clinical study as described hereinafter.

### In vivo test in rats for blood glucose control

Male Zucker fa/fa rats (Charles River, PA) fed standard rodent chow were housed individually in a room with a reverse light cycle (8:00 pm-8:00 am). The rats, 9-11 weeks of age, were cannulated in the right jugular vein 5-7 days prior to initiation of dosing. The animals were orally dosed once a day for 21 days with vehicle solution (0.5% methylcellulose; n=6), test compounds alone [NVP-LAF237 (10 umole/kg; n=7) or micronized fenofibrate (100 mg/kg; n=8)] or the combination (NVP-LAF237 and micronized fenofibrate). On the 21^{st} day, oral glucose tolerance test was performed. The rats were fasted for 18 hours before the test. Test compounds were given immediately after the baseline sample was taken at 15 minutes prior to glucose load. Glucose at 1 mg/kg was orally dosed to animals and blood samples were taken at 0, 5,10, 15, 20, 30, 45, 60 and 90 minutes after dose. The blood samples were immediately transferred into chilled tubes (containing EDTA and trasylol solution) and centrifuged. Plasma samples were isolated and stored at -20°C for the future determination of glucose, insulin, DPP-IV, fatty acid and triglyceride concentrations. Data were expressed as mean ± SEM for each group. Two-way ANOVA analysis was performed. Our results clearly show:
- the OGTT glucose excursion improvement in animals which were orally dosed once a day for 21 days with vehicle solution (0.5% methylcellulose; n=6), test compounds alone [NVP-LAF237 (10 umole/kg; n=7) or micronized fenofibrate (100 mg/kg; n=8)] or the combination (NVP-LAF237 and micronized fenofibrate). The plasma glucose AUC (air under the curve) is reduced by 18% after administration of LAF237 alone, 7% after administration of micronized fenofibrate alone and 33% after administration of the combination LAF237 and micronized fenofibrate.
- the OGTT insulin sensitivity improvement (by decreasing insulin resistance) in animals which were orally dosed once a day for 21 days with vehicle solution (0.5% methylcellulose; n=6), test compounds alone [NVP-LAF237 (10 umole/kg; n=7) or micronized fenofibrate (100 mg/kg; n=8)] or the combination (NVP-LAF237 and micronized fenofibrate).
- the reduction in body weight gain in animals which were orally dosed once a day for 21 days with vehicle solution (0.5% methylcellulose; n=6), test compounds alone [NVP-LAF237 (10 umole/kg; n=7) or micronized fenofibrate (100 mg/kg; n=8)] or the combination (NVP-LAF237 and micronized fenofibrate).

The combination treatment of NVP-LAF237 and micronized fenofibrate significantly improved OGTT glucose, improved insulin sensitivity and reduced body weight gain as compared to vehicle or either test compound alone.

### Clinical double-blind, randomized, Darallel-group study in subjects with type 2 diabetes mellitus inadequately controlled on diet alone

This study proves, in particular, the benefits of the claimed combined preparation or pharmaceutical composition, respectively. The beneficial effects on conditions mediated by DPP-IV, in particular type 2 diabetes mellitus can be determined directly through the results of this study or by changes in the study design which are known as such to a person skilled in the art.

The study is, in particular, suitable to compare the effects of monotherapy with a COMBINATION PARTNER OF THE INVENTION with those of a combination of DPP-IV inhibitor plus one of these compounds on glycemic control.

Subjects with a diagnosis of type 2 diabetes mellitus who have not achieved near normoglycemia (HbA_{1c} <6.8%) on diet only are chosen for this trial. The effects on glycemic control achieved with DPP-IV monotherapy, monotherapy with one COMBINATION PARTNER OF THE INVENTION, and the combination therapy of DPP-IV plus one COMBINATION PARTNER OF THE INVENTION are determined in this study after 24 weeks with the control achieved on placebo, all subjects continuing with the same diet as in the period before treatment. Measures of glycemic control are validated surrogate endpoints for the treatment of diabetes. HbA_{1c} is the single most reliable measurement for assessing glycemic control (see Goldstein et al., "Tests of Glycemia in Diabetes", Diabetes Care, Vol. 18, No. 6, pp. 896-909 (1995)) and is the primary response variable in this study. Since glycosylation of hemoglobin is determined by the glucose concentration at the time each red blood cell is made, HbA_{1c} provides an estimate of mean blood glucose for the previous three months.

The subjects are then separated into four treatment groups for the 24-week double-blind study (period II) as depicted in Tables 1-3 for the case that LAF237 is chosen as the DPP-IV inhibitor and one of the drugs comprising the micronized fenofibrate, bezafibrate or **gemfibrozil** is chosen as the combination partner.

### Examples for Combinations to be administered

**Table 1. LAF 237 Plus Micronized Fenofibrate**

| |
|---|
| LAF 237 10 mg* + micronized fenofibrate placebo** |
| Micronized fenofibrate 200 mg** + LAF237 placebo* |
| LAF237 10 mg* + micronized fenofibrate 200 mg** |
| LAF237 placebo* + micronized fenofibrae placebo** |

| |
|---|
| *Administered before breakfast, lunch and dinner. **Administered once daily with breakfast. |

**Table 2. LAF237 Plus Bezafibrate**

| |
|---|
| LAF237 10 mg* + bezafibrate placebo** |
| Bezafibrate 400 mg** + LAF237 placebo* |
| LAF237 10 mg* + bezafibrate 400 mg** |
| LAF237 placebo* + bezafibrate placebo** |

| |
|---|
| *Administered before breakfast, lunch and dinner. **Administered once daily with breakfast. |

**Table 3. LAF237 Plus gemfibrozil**

| |
|---|
| LAF237 10 mg* + gemfibrozil placebo** |
| gemfibrozil 600 mg** + LAF237 placebo* |
| LAF237 10 mg* + gemfibrozil 600 mg** |
| LAF237 placebo* + gemfibrozil placebo** |

| |
|---|
| *Administered before breakfast, lunch and dinner. **Administered twice a day with breakfast and dinner. |

LAF237 tablets contain either 10 mg of the compound or matching placebo. Micronied fenofibrate tablets contain either 200 mg or matching placebo. Micronized fenofibrate 200 mg tablets, bezafibrate 400 mg tablets and gemfibrozil 600 mg tablets can be purchased commercially and over-encapsulated to match the corresponding placebo capsules. In another very preferred embodiment, the inventions concerns combinations as described in the above tables containing 50 mg of LAF237.

The subjects are then separated into four treatment groups for the 24-week double-blind study (period II) as depicted in Table 1. Approximately 170 subjects are randomized per treatment group. The total study duration including the run-in period for each subject is 28 weeks. Statistical analysis can be carried out by methods known in the art.

The subject is advised not to take the morning dose of study medication or eat breakfast on the day of a scheduled study visit. The morning dose is administered by site personnel after the collection of all fasting laboratory samples and completion of all study procedures. Visits are scheduled to be performed at 2 week intervals during period I, and 4-8 week intervals during period 11. Subjects have fasted for at least 7 hours at the time of each visit. All blood samples for laboratory evaluations are drawn between 7:00 AM and 10:00 AM. All tests are conducted in accordance with Good Laboratory Practice principles following procedures known in the art.

HbA_{1c} is measured by High Performance Liquid Chromatography (HPLC) using the ionexchange method on a Bio-Rad Diamat analyzer. A back-up affinity method are used if hemoglobin variants or hemoglobin degradation peaks are observed.

Further parameters to be determined are fasting plasma glucose (FPG), fasting lipids (total, high density lipoprotein (HDL)- and low density lipoprotein (LDL)-cholesterol and triglycerides) and body weight. FPG will be measured using the hexokinase method and LDL-cholesterol will be calculated using the Friedewald formula if triglycerides are <400 mg/dL (4.5 mmol/L).

Various parameters of the study described above can be modified, e.g., in order to optimize the dosage for special diseases or indications mentioned herein, to cope with tolerability problems during the study or to obtain similar or identical results with less efforts. For example, a different subject population can be involved in such a clinical trial, e.g., subjects with a diagnosis of type 2 diabetes mellitus who have achieved near normoglycemia (HbA_{1c} <6.8%) on diet alone, subjects with diseases other than diabetes mellitus, e.g., other metabolic disorders, or subjects selected by other criteria, such as age or sex; the subject number can be decreased, e.g., to a number of between 70 and 150, especially 100 or 120, subjects per treatment group; treatment groups (listed exemplary in Table 1) can be deleted, i.e., for example to carry out a study with a comparison of the combination of a DPP-IV inhibitor and at least one further COMBINATION PARTNER OF THE INVENTION versus a DPP-IV inhibitor alone; the term of the placebo run-in period (period I) can be changed, i.e., it can be extended, shortened or deleted; the visit schedule can be extended, e.g., to every 10, 12 or 14 weeks; the visit instructions can be changed, e.g., the instruction that blood samples for laboratory evaluations have to be drawn between 7:00 AM and 10:00 AM; HbA_{1c} can be determined by other means; or one or more of the parameters to be determined during the study mentioned above, e.g., FPG or fasting lipids, can be deleted or the determination of additional parameters (see below) can be added.

Additional parameters can be determined in the course of the study, e.g., by additional tests. Such additional tests can comprise the analysis of body liquids in order to determine amounts or numbers for parameters, such as those listed below, and can serve, e.g., the purpose of determining the tolerability of the administered active ingredients:
- determination of hematocrit and hemogloblin, platelet count, erythrocyte count, total and differential leukocyte count, e.g., basophils, eosinophils, lymphocytes, monocytes, segmented neutrophils and total neutrophils;
- determination of albumin, alkaline phosphatase, alanine amino transferase, e.g., serum glutamic pyruvic transaminase, aspartate amino transferase, e.g., serum glutamic oxaloacetic transaminase, blood urea nitrogen or urea, bicarbonate, calcium, chloride, total creatine phosphokinase (CPK), creatine phosphokinase muscle-brain fraction isoenzyme (if CPK is elevated), direct bilirubin, creatinine, γ-glutamyl transferase, lactate dehydrogenase, potassium, sodium, total bilirubin, total protein and uric acid in the blood;
- determination of bilirubin, glucose, ketones, pH, protein and specific gravity in the subjects urine; and
- determination of body weight, blood pressure, e.g., systolic and diastolic, after 3 minutes sitting, and radial pulse (after 3 minutes sitting).

The results of the studies show that the combination according to the present invention can be used for the prevention and preferably the treatment of conditions mediated by DPP-IV or PPARα, in particular type 2 diabetes mellitus and dyslipidemia. The combination of the present invention can also be used for the prevention and preferably the treatment of other condition mediated by DPP-IV or PPARα.

Furthermore, in a number of combinations as disclosed herein the side effects observed with one of the components surprisingly do not accumulate on application of the combination.

Preferably, the jointly therapeutically effective amounts of the DPP-IV inhibitor (*S*)-1-[(3-hydroxy-1-adamantyl)amino]acetyl-2-cyano-pyrrolidine, in free or pharmaceutically acceptable salt form and PPARα compound are administered simultaneously or sequentially in any order, separately or in a fixed combination.

The condition mediated by DPP-IV or PPARα is preferably selected from the group consisting of diabetes, impaired fasting plasma glucose, impaired glucose tolerance, metabolic acidosis, ketosis, arthritis, obesity, dyslipidemia and osteoporosis.

Very preferably, the condition mediated by DPP-IV is type 2 diabetes mellitus and the condition mediated by PPARα is dyslipidemia.

It is one objective of this invention to provide a pharmaceutical composition comprising a quantity, which is jointly therapeutically effective against conditions mediated by DPP-IV or PPARα, in particular diabetes, more especially type 2 diabetes mellitus, dyslipidemia, conditions of impaired fasting plasma glucose, and conditions of IGT, of the DPP-IV inhibitor (*S*)-1-[(3-hydroxy-1-adamantyl)amino]acetyl-2-cyano-pyrrolidine: (i) or a pharmaceutically acceptable salt thereof; and (ii) at least one further COMBINATION PARTNER OF THE INVENTION and at least one pharmaceutically acceptable carrier.

The pharmaceutical compositions according to the invention can be prepared in a manner known *per se* and are those suitable for enteral, such as oral or rectal, and parenteral administration to mammals (warm-blooded animals), including man, comprising a therapeutically effective amount of the pharmacologically active compound, alone or in combination with one or more pharmaceutically acceptable carries, especially suitable for enteral or parenteral application.

The novel pharmaceutical preparations contain, for example, from about 10% to about 100%, e.g., 80% or 90%, preferably from about 20% to about 60%, of the active ingredient. Pharmaceutical preparations according to the invention for enteral or parenteral administration are, e.g., those in unit dose forms, such as sugar-coated tablets, tablets, capsules or suppositories and furthermore ampoules. These are prepared in a manner known *per se,* e.g., by means of conventional mixing, granulating, sugar-coating, dissolving or lyophilizing processes. Thus, pharmaceutical preparations for oral use can be obtained by combining the active ingredient with solid carriers, if desired granulating a mixture obtained, and processing the mixture or granules, if desired or necessary, after addition of suitable excipients to give tablets or sugar-coated tablet cores.

In this composition, components (i) and (ii) can be administered together, one after the other or separately in one combined unit dose form or in two separate unit dose forms. In one preferred embodiment of the invention, the unit dose form is a fixed combination. In a fixed combination the components (i) and (ii) are administered in the form of a single galenic formulation, e.g., a single tablet or a single infusion.

A further aspect of the present invention is the use of a pharmaceutical composition comprising the DPP-IV inhibitor (*S*)-1-[(3-hydroxy-1-adamantyl)amino]acetyl-2-cyano-pyrrolidine, and at least one further COMBINATION PARTNER OF THE INVENTION, in each case in free form or in form of a pharmaceutically acceptable salt thereof for the preparation of a pharmaceutical preparation for the prevention or treatment of conditions mediated by DPP-IV or PPARα, in particular diabetes, more especially type 2 diabetes mellitus, conditions of impaired fasting plasma glucose, dyslipidemia and conditions of IGT.

A therapeutically effective amount of each of the components of the combination of the present invention may be administered simultaneously or sequentially and in any order, and the components may be administered separately or as a fixed combination. For example, the method of treatment of the invention may comprise: (i) administration of the DPP-IV inhibitor (*S*)-1-[(3-hydroxy-1-adamantyl)amino]acetyl-2-cyano-pyrrolidine, in free or pharmaceutically acceptable salt form; and (ii) administration of at least one further COMBINATION PARTNER OF THE INVENTION simultaneously or sequentially in any order, in jointly therapeutically effective amounts, preferably in synergistically effective amounts, e.g., in daily dosages corresponding to the ratios described herein.

The corresponding active ingredient or a pharmaceutically acceptable salt thereof may also be used in form of a hydrate or include other solvents used for crystallization.

The invention relates in particular to a commercial package comprising jointly therapeutically effective amounts of the DPP-IV inhibitor (*S*)-1-[(3-hydroxy-1-adamantyl)amino]acetyl-2-cyano-pyrrolidine, in free or pharmaceutically acceptable salt form, and at least one further COMBINATION PARTNER OF THE INVENTION together with instructions for use thereof in the treatment of conditions mediated by DPP-IV or PPARα, in particular diabetes, more especially type 2 diabetes mellitus, conditions of impaired fasting plasma glucose, dyslipidemia and conditions of IGT.

The present invention furthermore concerns;
1) A combination according to the present invention for use as a medicament.
2) The use of the dipeptidylpeptidase-IV (DPP-IV) inhibitor (*S*)-1-[(3-hydroxy-1-adamantyl)amino]acetyl-2-cyano-pyrrolidine, in free or pharmaceutically acceptable salt form, in combination with at least one further COMBINATION PARTNER OF THE INVENTION for the manufacture of a medicament for the treatment a condition mediated by DPP-IV or PPARα, which is selected from type 2 diabetes mellitus, conditions of IGT, conditions of impaired fasting plasma glucose, obesity, and dyslipidemia.
3) The use of the dipaptidylpeptidase-IV (DPP-IV) inhibitor (*S*)-1-[(3-hydroxy-1-adamantyl)amino]acetyl-2-cyano-pyrrolidine, or pharmaceutically acceptable salt form in combination with at least one further PPARα compound selected from the group consisting of fenofibrate, micronized fenofibrate, bezafibrate, gemfibrozil and ciprofibrate, or the pharmaceutically acceptable salt of such a compound for the manufacture of a medicament for the treatment a condition mediated by DPP-IV or PPARα, in particular type 2 diabetes, impaired glucose tolerance, obesity and dyslipidemia.
4) The use of a (*S*)-1-[(3-hydroxy-1-adamantyl)amino]acetyl-2-cyano-pyrrolidine
   or pharmaceutically acceptable salt form in combination with at one further PPARα compound selected from the group consisting of fenofibrate, micronized fenofibrate, bezafibrate, gemfibrozil and ciprofibrate, or the pharmaceutically acceptable salt of such a compound INVENTION for the manufacture of a medicament for the treatment a condition mediated by DPP-IV or PPARα, which is selected from type 2 diabetes, impaired glucose tolerance, obesity and dyslipidemia.
5) Use of a combination according to the present invention for the manufacture of a medicament for the treatment a condition mediated by DPP-IV or PPARα, which is selected from, type 2 diabetes mellitus, conditions of IGT, conditions of impaired fasting plasma glucose, obesity, and dyslipidemia.

The dosage range of the combination of a DPP-IV inhibitor and at least one further COMBINATION PARTNER OF THE INVENTION to be employed depends upon factors known to the person skilled in the art including species of the warm-blooded animal, body weight and age, the nature and severity of the condition to be treated, the mode of administration and the particular substance to be employed. Unless stated otherwise herein, the DPP-IV inhibitor and at least one further COMBINATION PARTNER OF THE INVENTION are preferably divided and administered from one to four times per day.

The weight ratio of the daily doses of LAF237 or a pharmaceutically acceptable salt thereof to at least one further COMB1NATION PARTNER OF THE INVENTION may vary within wide limits depending, in particular, on the needs of the warm-blooded animal treated.

## Claims

1. Use of a DPP-IV inhibitor which is (S)-1-[(3-hydroxy-1-adamantyl)amino]acetyl-2-cyano-pyrrolidirte, in free or pharmaceutically acceptable salt form, in combination with at least one further PPARα compound, or the pharmaceutically acceptable salt of such a compound,
for the manufacture of a medicament for the prevention, delay of progression or treatment of a disease selected from type 2 diabetes, impaired glucose tolerance, obesity and dyslipidemia.

2. Use according to claim 1 for the manufacture of a medicament for the prevention, delay of progression or treatment of type 2 diabetes.

3. Use according to claim 1 for the manufacture of a medicament for the prevention, delay of progression or treatment of dyslipidemia.

4. Use according to any of claims 1 to 3 wherein the further PPARα compound is selected from the group consisting of fenofibrate, micronized fenofibrate, bezafibrate, gemfibrozil and ciprofibrate, or the pharmaceutically acceptable salt of such a compound.

5. A combination, which comprises the DPP-IV inhibitor (S)-1-[(3-hydroxy-1-adamantyl)amino]acetyl-2-cyano-pyrrolidine, in free or pharmaceutically acceptable salt form; and at least one further PPARα compound or the pharmaceutically acceptable salt of such a compound.

6. A pharmaceutical combination comprising the DPP-IV inhibitor (*S*)-1-[(3-hydroxy-1-adamantyl)amino]acetyl-2-cyano-pyrrolidine, in free form or in acid addition salt form; and at least one further PPARα compound or the pharmaceutically acceptable salt of such a compound, and optionally at least one pharmaceutically acceptable carrier; for simultaneous, separate or sequential use.

7. A combination according to claim 5 or claim 6, which is a combined preparation.

8. A combination according to claim 5 or claim 6, which is a fixed combination.

9. A combination according to any of claims 5 to 8, wherein the further PPARα compound is selected from the group consisting of fenofibrate, micronized fenofibrate, bezafibrate, gemfibrozil and ciprofibrate, or the pharmaceutically acceptable salt of such a compound.

10. A combination according to any one of claims 5 to 9 for use as a medicament.

## Patentansprüche

1. Verwendung eines DPP-IV-Inhibitors, der (S)-1-[(3-Hydroxy-1-adamantyl)-amino]-acetyl-2-cyano-pyrrolidin ist, in freier Form oder in Form eines pharmazeutisch akzeptablen Salzes, in Kombination mit wenigstens einer weiteren PPARa-Verbindung, oder einem pharmazeutisch akzeptablen Salze einer solchen Verbindung, zur Herstellung eines Arzneimittels für die Prävention, Progressionsverzögerung oder Behandlung einer Krankheit, die ausgewählt ist aus Diabetes Typ 2, gestörter Glucosetoleranz, Obesität und Dyslipidämie.

2. Verwendung nach Anspruch 1 zur Herstellung eines Arzneimittels für die Prävention, Progressionsverzögerung oder Behandlung von Diabetes Typ 2.

3. Verwendung nach Anspruch 1 zur Herstellung eines Arzneimittels für die Prävention, Progressionsverzögerung oder Behandlung von Dyslipidämie.

4. Verwendung nach einem der Ansprüche 1 bis 3, worin die weitere PPARα-Verbindung aus der Gruppe ausgewählt ist, die besteht aus Tenofibrat, mikronisiertem Tenofibrat, Bezafibrat, Gemfibrozil und Ciprofibrat, oder einem pharmazeutisch akzeptablen Salz einer solchen Verbindung.

5. Kombination, umfassend den DPP-IV-Inhibitor (S)-1-[(3-Hydroxy-1-adamantyl)-amino]-acetyl-2-cyanopyrrolidin, in freier Form oder in Form eines pharmazeutisch akzeptablen Salzes und wenigstens eine weitere PPARα-Verbindung oder ein pharmazeutisch akzeptables Salz einer solchen Verbindung.

6. Pharmazeutische Kombination, umfassend den DPP-IV-Inhibitor (S)-1-[(3-Hydroxy-1-adamantyl)-amino]-acetyl-2-cyanopyrrolidin, in freier Form oder in Form eines Säureadditionssalzes, und wenigstens eine weitere PPARα-Verbindung oder ein pharmazeutisch akzeptables Salz einer solchen Verbindung, und optional wenigstens einen pharmazeutisch akzeptablen Träger, für die simultane, separate oder sequenzielle Anwendung.

7. Kombination nach Anspruch 5 oder 6, die ein Kombinationspräparat ist.

8. Kombination nach Anspruch 5 oder 6, die eine fixe Kombination ist.

9. Kombination nach einem der Ansprüche 5 bis 9, worin die weitere PPARα-Verbindung ausgewählt ist aus der Gruppe, die besteht aus Tenofibrat, mikronisiertem Tenofibrat, Bezafibrat, Gemfibrozil und Ciprcfibrat, oder einem pharmazeutisch akzeptablen Salz einer solchen Verbindung.

10. Kombination nach einem der Ansprüche 5 bis 9 für die Verwendung als ein Arzneimittel.

## Revendications

1. Utilisation d'un inhibiteur de la DPP-IV qui est la (S)-1-[(3-hydroxy-1-adamantyl)amino]acétyl-2-cyano-pyrrolidine, sous forme libre ou sous la forme d'un sel acceptable sur le plan pharmaceutique, en combinaison avec au moins un composé supplémentaire PPARα, ou le sel acceptable sur le plan pharmaceutique d'un tel composé, pour fabriquer un médicament destiné à prévenir, retarder l'évolution ou traiter une maladie sélectionnée parmi le diabète de type 2, l'intolérance au glucose, l'obésité et la dyslipidémie.

2. Utilisation selon la revendication 1 pour la fabrication d'un médicament destiné à prévenir, retarder l'évolution ou traiter le diabète de type 2.

3. Utilisation selon la revendication 1 pour la fabrication d'un médicament destiné à prévenir, retarder l'évolution ou traiter la dyslipidémie.

4. Utilisation selon l'une quelconque des revendications 1 à 3, dans laquelle le composé supplémentaire PPARα est sélectionné dans le groupe constitué par le fénofibrate, le fénofibrate micronisé, le bézafibrate, le gemfibrozil et le ciprofibrate, ou le sel acceptable sur le plan pharmaceutique d'un tel composé.

5. Combinaison, qui comprend l'inhibiteur de la DPP-IV qui est la (S)-1-[(3-hydroxy-1-adamantyl)amino]acétyl-2-cyana-pyrrolidine, sous forme libre ou sous la forme d'un sel acceptable sur le plan pharmaceutique ; et au moins un composé supplémentaire PPARα ou le sel acceptable sur le plan pharmaceutique d'un tel composé.

6. Combinaison pharmaceutique comprenant l'inhibiteur de la DPP-IV qui est la (S)-1-[(3-hydroxy-1-adamantyl)amino]acétyl-2-cyano-pyrrolidine, sous forme libre ou sous la forme d'un sel d'addition d'acide ; et au moins un composé supplémentaire PPARα ou le sel acceptable sur le plan pharmaceutique d'un tel composé, et éventuellement au moins un support acceptable sur le plan pharmaceutique ; pour une utilisation simultanée, distincte ou séquentielle.

7. Combinaison selon la revendication 5 ou la revendication 6, qui est une préparation combinée.

8. Combinaison selon la revendication 5 ou la revendication 6, qui est une combinaison fixe.

9. Combinaison selon l'une quelconque des revendications 5 à 8, dans laquelle le composé supplémentaire PPARα est sélectionné dans le groupe constitué par le fénofibrate, le fénofibrate micronisé, le bézafibrate, le gemfibrozil et le ciprofibrate, ou le sel acceptable sur le plan pharmaceutique d'un tel composé.

10. Combinaison selon l'une quelconque des revendications 5 à 9 pour une utilisation en tant que médicament.
